(19) **Europäisches Patentamt / European Patent Office / Office européen des brevets**

(11) **EP 2 035 831 B1**

(12) **EUROPEAN PATENT SPECIFICATION**

(45) Date of publication and mention
of the grant of the patent:
**14.08.2013 Bulletin 2013/33**

(51) Int Cl.:
*G01N 33/53* (2006.01)  *C12Q 1/68* (2006.01)
*G01N 33/487* (2006.01)  *G01N 33/566* (2006.01)

(21) Application number: **07812152.2**

(22) Date of filing: **14.06.2007**

(86) International application number:
**PCT/US2007/071270**

(87) International publication number:
**WO 2008/005674 (10.01.2008 Gazette 2008/02)**

(54) **METHODS OF ANALYZING BINDING INTERACTIONS**

VERFAHREN ZUR ANALYSE VON VERBINDUNGSINTERAKTIONEN

PROCÉDÉS D'ANALYSE D'INTERACTIONS DE LIAISON

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **30.06.2006 US 817956 P**

(43) Date of publication of application:
**18.03.2009 Bulletin 2009/12**

(73) Proprietor: **Applied Biosystems, LLC**
**Carlsbad, CA 92008 (US)**

(72) Inventors:
• **SUN, Hongye**
**Belmont, CA 94002 (US)**
• **LIVAK, Kenneth J.**
**San Jose, CA 95117 (US)**

(74) Representative: **Wöhler, Christian**
**Life Technologies**
**Frankfurter Straße 129 B**
**64293 Darmstadt (DE)**

(56) References cited:
WO-A-2005/017025    WO-A2-2007/120265
US-A1- 2007 247 170    US-B1- 6 428 959

• **TOBIAS AMBJÄRNSSON ET AL: "Directed motion emerging from two coupled random processes: translocation of a chain through a membrane nanopore driven by binding proteins; Directed motion emerging from two coupled random processes: translocation of a chain through a membrane nanopore driven by binding proteins" JOURNAL OF PHYSICS: CONDENSED MATTER, INSTITUTE OF PHYSICS PUBLISHING, BRISTOL, GB, vol. 17, no. 47, 30 November 2005 (2005-11-30), pages S3945-S3964, XP020089717 ISSN: 0953-8984**

**Description**

## 1. INTRODUCTION

Binding interactions between a ligand and its cognate binding partner are critical for many chemical and biological processes. For instance, interactions between biological macromolecules, such as between a protein and a DNA or between one protein and another protein, form the critical regulatory networks for controlling cell development and cell activity. For some binding interactions, such as the interaction between a ligand and its corresponding cellular receptor, it is desirable to determine the strength of interaction between the receptor and ligand since the time of receptor occupancy can regulate the strength of the corresponding biological response. More complex binding interactions, such as cooperativity between binding sites, can exquisitely regulate the ensuing biological event. For example, the decision between lytic and lysogenic phases of bacteriophage $\lambda$ depends, in part, on the activity of $\lambda$ repressor protein *cI*, which binds cooperatively to a tripartite repressor binding site. Because of the cooperative nature of *cI* repressor binding to its operator sites, minor changes in the level of repressor protein can trigger the phage into the lytic mode from the lysogenic mode and vice versa. Consequently, understanding complex binding phenomena, such as cooperative and allosteric binding interactions, can assist in studying how biological systems regulate certain processes.

A number of different methods are available for ascertaining the binding characteristics between interacting molecules. One common solution-based approach is equilibrium dialysis, a technique in which a receptor component is entrapped by a membrane that is impermeable to the receptor but permeable to the ligand. Labeled ligand is used to determine the amount of ligand bound to the receptor at different ligand concentrations, which can provide sufficient information to estimate the equilibrium binding constant, the number of interaction sites for the ligand, and the presence or absence of cooperative interactions. Other approaches to studying binding interactions include chromatography and sedimentation analysis. Chromatographic approaches examine the elution behavior of the interacting mixture when the mixture is separated in a chromatographic medium (*e.g.*, molecular sieve) while sedimentation analysis examines the equilibrium sedimentation behavior of the mixtures when subjected to high centrifugal forces. In some instances, spectroscopic techniques in which the absorption and/or emission characteristics of free and bound components are distinguishable provide another approach for analysis. Example using spectroscopic techniques are the hyperchromicity shift used to detect the annealing/denaturation of one polynucleotide to another polynucleotide, and fluorescence resonance energy transfer (FRET) between donor and acceptor chromophores attached to interacting molecules.

Although the forgoing techniques can generate useful measures of the interaction between molecules, these techniques can require significant amount of samples for analysis, which may not be suitable for studying interactions where the binding and receptor components are not readily available or where large number of binding interactions must be assessed. Spectroscopic techniques are also limited to molecules that have the requisite spectral properties. If the spectroscopic technique uses detectable labels, the interacting components must be appropriately labeled, which can be problematic if the binding activity of the labeled component is sensitive to the modification.

More sensitive techniques for ascertaining the binding characteristics of two interacting molecules employ attachment of one of the interacting molecules to a surface and detection of a signal associated with the binding of a ligand to the surface bound molecule. Although the ligand or receptor can be tagged with a detectable label for detecting the bound complexes, labels can be avoided where detection of the binding event relies on changes to a measurable property of the surface material. Examples of such properties include, among others, reflection of circularly polarized light of a gold layer (*i.e.*, surface plasmon resonance) or electrical conductivity of an electrode surface (see, *e.g.*, Myszka, D., 2000, Methods Enzymol. 323:325-340).

Although analysis of binding interactions on a surface is a sensitive technique and can simplify the measurement of binding parameters, and where appropriate, avoids use of detectable labels, the surface effects on the binding events can complicate analysis of binding interactions. For example, if the mass transport of the ligand to the surface is slow relative to the rate of binding, deviation from typical binding characteristics observed in solution can occur. Moreover, the bound receptor can affect the kinetics of interaction with the ligand since it is not free to diffuse, thereby adding another factor for deviation from solution behavior.

Thus, it is desirable to find alternative techniques useful for examining binding interactions that bypass the use of labels and avoid surface binding effects while maintaining high sensitivity.

## 2. DETAILED DESCRIPTION

It is to be understood that the following detailed description are exemplary and explanatory only and are not restrictive of this disclosure. In this disclosure, the use of the singular includes the plural unless specifically stated otherwise. Also, the use of "or" means "and/or" unless stated otherwise. Similarly, "comprise," "comprises," "comprising" "include," "includes," and "including" are interchangeable and not intended to be limiting.

It is to be further understood that where descriptions of various embodiments use the term "comprising," those skilled in the art would understand that in some specific instances, an embodiment can be alternatively described using language "consisting essentially of" or "consisting of."

The section headings used herein are for organizational purposes only and not to be construed as limiting the subject matter described.

2.1 Binding Interactions and Methods for Analysis of Binding Interactions

The present disclosure provides methods of analyzing binding interactions between at least two components in solution. The methods herein are based on the ability to detect and distinguish the various components in a binding mixture by passing the mixture through a detection region and quantitating the various unbound and bound forms present in the mixture. In the embodiments herein, the detection region is part of a pore or channel through which the interacting components are translocated. Based on differences in the detectable properties of the unbound and bound components, the number of binding components bound per number of receptor component can be determined. This measurement provides sufficient information to assess the various parameters of a binding interaction, such as equilibrium binding constants, association and dissociation rates, as well as interaction between multiple sites on the receptor component. Because the binding interactions are carried out in solution rather than on a surface, the methods avoid the complications associated with binding reactions carried out on a surface.

Generally, the method of analyzing binding interactions comprises contacting one or more binding components and a receptor component in solution, wherein unbound and any bound components are capable of being translocated through a pore. After forming the mixture, the components in the mixture are translocated through a suitably sized pore and detected using a suitable detection technique, as further described below. In various embodiments, the pore comprises a detection region for detecting components translocated through the pore. Different components in the mixture can be quantitated if the detected signal pattern associated with each component is distinguishable from the signal patterns observed for other components in the binding mixture. Various parameters reflecting the binding interactions can be assessed by determining the number of binding component bound per number of receptor component.

As used herein, "binding interaction" refers to any physical association between one component and another component. Interactions can include covalent interactions and non-covalent interactions. Non-covalent interactions can involve, among others, hydrophobic interactions, dipole-dipole interactions, van der Waals forces, hydrogen bonding, ionic interactions, and combinations thereof. Interacting components can be any molecule or composition. "Specific" binding interactions refer to the binding of a binding component to a specific interaction site on a receptor molecule such that the interaction displays saturable binding behavior. Generally, concentration of binding component at which the interaction sites are 50% saturated is referred to as the equilibrium binding constant $K_{eq}$. The inverse of the equilibrium binding constant is the equilibrium dissociation constant ($K_{diss}$ or $K_{ds}$). $K_{ds}$ for specific interactions can range from about $10^{-2}$ M or less, about $10^{-4}$ M or less, $10^{-7}$ M or less, or $10^{-10}$ M or less, or about $10^{-14}$ M or less. In addition to specific binding interactions, some binding interactions are non-specific in nature. As used herein, "non-specific" interaction refers to binding interactions in which the binding of a binding component to a receptor component is linearly proportional to the concentration of binding component, and therefore does not show saturable binding under the conditions where saturation of specific binding sites are observed. Binding interactions can comprise a mixture of specific and non-specific interactions.

The term "binding component" refers to any compound or composition that is capable of associating with a receptor component in a binding interaction. The binding component can comprise, by way of example and not limitation, a small organic molecule ligand; polypeptides and polypeptide analogs; nucleobase polymers, such as polynucleotides and polynucleotide analogs; carbohydrates, such as monosaccharides, oligosaccharides, and polysaccharides; and lipids and related fatty acids. In some embodiments, the binding component can be a single molecule, such as a single protein or oligonucleotide, or comprise a complex of molecules that together form a binding component. Examples of binding component complexes include, among others, protein-protein, small molecule-protein, carbohydrate-protein, protein-polynucleotide, and small molecule-nucleobase polymer complexes. For example, DNA binding proteins are known to dimerize to form an active DNA binding complex capable of binding to a specific sequence on a polynucleotide.

The term "receptor component" refers to any compound or composition that is capable of associating with a binding component in a binding interaction. The receptor component can comprise a small organic molecule; polypeptides and polypeptide analogs; nucleobase polymers, such as polynucleotides and polynucleotide analogs; carbohydrates, such as monosaccharides, oligosaccharides, and polysaccharides; and lipids and related fatty acids. In some embodiments, the receptor component can be a single molecule, such as a single protein or oligonucleotide, or comprise a complex of molecules that together form a receptor component. Examples of a receptor component complex, include, among others, protein-protein, small molecule-protein, carbohydrate-protein, protein-polynucleotide, and small molecule-polynucleotide complexes. For example, the estrogen receptor comprise dimers of $\alpha$ and $\beta$ subunits, and thus can form various receptors subtypes, including, $\alpha\alpha$, $\beta\beta$ and $\alpha\beta$ dimers. Various binding and receptor components are described below.

The term "interaction site" refers to any site on the receptor component that is capable of interacting with the binding component. In some embodiments, the interaction site can be on the surface, or the inner portion of the receptor component, accessible or inaccessible to solution. Although in some embodiments binding interactions necessitate accessibility of the interaction site to the solution, an inaccessible interaction site can be made accessible to solution by the action of other binding components (*e.g.*, allosteric compounds). Interactions sites can be present on a single receptor component or be generated through formation of receptor component complexes. In various embodiments, the number of interaction sites present on the receptor component can be one or more than one, as further described below.

It is to be understood that description of a component as a binding or receptor component are not meant to be limiting since, in some instances, a binding component can be described as a receptor component and vice versa.

Upon contacting the binding components with the receptor component, the mixture can comprise unbound components and, where binding interactions occur, bound components. "Unbound component" refers to a binding component or receptor component that is not bound to the other component. Thus, the unbound component is binding component and receptor component existing free in solution. "Bound component" refers to a binding component that is physically interacting or associated with a receptor component, thereby being in the form of a complex.

Different types of binding interactions have been characterized and described. The binding interactions between a binding component (L) and a receptor component (M), where a single interaction site is present on the receptor component, can be described by the following reaction:

$$M + L = ML$$

In the binding interaction above, M and L represent the unbound components while ML represents the bound component. The equilibrium binding constant for the reaction is represented by:

$$K_{eq} = \frac{[ML]}{[M][L]}$$

The dissociation constant, $K_{ds}$, is the inverse of $K_{eq}$. For a receptor component with a single interaction site, the fractional saturation, $Y$, is the fraction of receptor component saturated with the binding component:

$$Y = \frac{[ML]}{[M]+[ML]}$$

The fractional saturation can be expressed with respect to the dissociation constant as follows:

$$Y = \frac{[L]}{K_{ds}+[L]}$$

Thus, ascertaining the concentration of bound component or the concentration of unbound binding components and/or unbound receptor component can provide sufficient information for determining the dissociation constant for the binding interaction involving a single interaction site. This type of reaction is generally referred to as the *Langmuir isotherm.* When the receptor component is half saturated with the binding component, the binding component concentration is equal to the dissociation constant $K_{ds}$.

For binding interactions in which more than one interaction site is present on the receptor component, and the interaction sites do not influence binding at other interaction sites, the binding interaction can be described with reference to $v$, which represents the moles of binding component bound per mole of receptor component. For a receptor component with $n$ number of interaction sites with the same $K_{ds}$ for each site, the binding interaction can be generalized to the following:

$$v = \frac{n[L]}{K_{ds} + [L]}$$

The equation above does not account for interactions in which binding of a first binding component to one site of the receptor component changes the binding of a second binding component to a second interaction site. Such communication between interaction sites includes, among others, cooperative interaction and allosteric regulation. As used herein, "cooperative interaction" and "allosteric regulation" refer to the binding of a first binding component to a first interaction site on a receptor component and its effect on the binding of a second binding component to a second interaction site. Cooperative interaction is a special form of allosteric regulation and refers to changes in affinity of a receptor component for a binding component, where the change in affinity is dependent on the amount of binding component already bound to the receptor component. Positive cooperativity is present where the binding of the first binding component results in a higher affinity of the second interaction site for binding to the second binding component. Negative cooperativity is present where the binding of the first binding component results in a lower affinity of the second interaction site for binding to the second binding component. In some embodiments, cooperative interactions can be represented by the following reaction:

$$M + nL = ML_n$$

where n is the number of interaction sites on the receptor component $M$. The average equilibrium binding constant for the reaction is:

$$K_n = \frac{[ML_n]}{[M][L]^n}$$

For a reaction in which there is infinite cooperativity, the value v, the moles of binding component bound per mole of receptor component, can be expressed as follows:

$$v = \frac{nK_n[L]^n}{1 + K_n[L]^n}$$

Since the fractional saturation $Y$ is $Y = v/n$, the formula above reduces to:

$$Y = \frac{[L]^n}{K_d + [L]^n}$$

where $K_{ds} = 1/K_n$. As noted above, the foregoing equation is based on infinite cooperativity. For less cooperative systems, the binding interactions can be described empirically by the following:

$$Y = \frac{K_n[L]^{n_h}}{1 + K_n[L]^{n_h}}$$

where $n_h$ is known as the Hill Coefficient. The Hill coefficient describes the degree of cooperative, where a value of 1 indicates non-cooperativity and a $n_h$ value of N (the total number of bound ligands) indicates infinite cooperativity. A value of $n_h > 1$ indicates negative cooperativity while a value of $n_h < 1$ indicates positive cooperativity.

As suggested from the descriptions of various binding interactions, binding parameters can be obtained by determining the number of binding components bound per number of receptor components. This can be done by determining the concentration of unbound ligand, unbound receptor component, and/or bound components in the reaction mixture. In some embodiments, determining the number of binding components bound per number of receptor component is carried out at different concentrations of binding component and receptor component. In other embodiments, determining the number of binding components bound per number of receptor components is carried out at different concentrations of binding components and constant (i.e., same) concentration of receptor component. For example, increasing concentrations of the binding component can be added to the system to determine the equilibrium binding constant. Low concentrations are in the range of the $K_{ds}$ while high concentrations are much higher and approach saturation of the binding site. Hence, the range of binding concentration measurements is often over several orders of magnitude. An exemplary method of determining the $K_{ds}$ using different concentrations of one of the components in the reaction is represented by the following equation:

$$[ML] = ([M]_T + [L]_T + K_d - \sqrt{([M]_T + [L]_T + K_d)^2 - 4[M]_T[L]_T}$$

As above, [ML] is the concentration of the bound components at the different concentrations of binding component $[L]_T$ and at fixed concentration of receptor component $[M]_T$. By determining the number of binding component bound per number of receptor component at different concentrations of [L], the concentration of bound component [ML] can be measured, thereby allowing calculation of the $K_{ds}$.

In some embodiments, the binding kinetics, such as the association rate constant ($k_{on}$) and dissociation rate constant ($k_{off}$), can also be examined using the methods herein. For example, the associative phase of a binding reaction can be assessed by rapid mixing of the binding component and receptor component, such as by injecting the components separately into a mixing chamber in fluid communication with the detection region. The mixture is then translocated through the pore and the detection region to determine the number of binding components bound per number of receptor component. Counting the number of complexes formed over time until attainment of equilibrium can be used to determine the association rate constant. For a reaction involving a receptor component with a single interaction site, the association kinetics can be expressed as:

$$\frac{d[ML]}{dt} = k_{on}[L][M] - k_{off}[ML]$$

where $d[A_1L]/dt$ is the change in the number of bound components as a function of time. At time $t$, [M] is given by $[M_{total}]$ - [ML]. The association rate can then be expressed as follows:

$$\frac{d[ML]}{dt} = k_a[L]\{[M_{total}] - [ML]\} - k_d[ML]$$

Measuring the time required from mixing to a point near or at equilibrium value of [ML] can be used to determine the association rate constant. To simplify the measurement and reduce the effect of dissociation, the measurements can be carried out under conditions where dissociation is negligible, such as before formation of significant amount of [ML] complexes. Where M and L represent non-identical components, determining the association rate constants can be simplified by (1) mixing M and L and estimating the initial rate at different concentrations of M and L, (2) carrying out the experiments under conditions in which [M] = [L], thereby mimicking a homogeneous dimerization reaction, or (3) measuring the rate where [L] is in vast excess of [M] such that [L] does not significantly change over time.

In some embodiments, the methods herein can be used to determine the dissociation rate constants. For example, the binding component and receptor components can be mixed to allow binding interactions to take place and then rapidly diluted to cause dissociation of the bound binding components from the receptor component. Carrying out the experiments under conditions in which the association reaction is negligible, such as measuring the dissociation reaction before significant reassociation of dissociated complexes or where the final diluted concentration is far below the $K_{eq}$, the rate equation simplifies to the following:

$$-\frac{d[ML]}{dt} = \frac{d[M]}{dt} = k_{off}[ML]$$

As such, the decrease in the number of binding component bound per number of receptor component as a function of time can be used to estimate the dissociation rate constant. Other methods of determining the rate constants will be apparent to the skilled artisan and are not to be limited to the embodiments disclosed herein.

In accordance with the above, a variety of binding interactions are amenable to analysis using the methods described herein. In some embodiments, the methods can be used to examine binding interactions in which the receptor component has a single interacting site (*i.e.*, number *n* of interacting site where *n* = 1), the basic equations of which have been described above. In other embodiments, the binding interactions can involve receptor components in which the *n* number of interaction sites is greater than 1. In some embodiments, the binding interactions examined can involve a receptor component in which the *n* number of interaction sites is 2 or more, 4 or more, 8 more, 10 or more, and 20 or more. In some embodiments, the binding interactions can involve receptor component in which the *n* number of interacting site is greater than 20, such as the binding of small molecules to polymers, for example, intercalator interactions with polynucleotides. In some embodiments, the binding interactions can involve receptor components with *n* number of interacting sites from 2 up to 10.

In some embodiments where the number of interacting sites is greater than 1, each of the interacting sites can comprise identical equilibrium binding constants. In other embodiments where the number of interacting sites is greater than 1, at least two of the interactions sites can comprise different equilibrium binding constants. Examples of these interactions include, among others, allosterically regulated enzymes and proteins. In some instances, all of the interaction sites can have different equilibrium binding constants, particularly in systems involving cooperative interactions. Cooperative interactions are seen in many biological binding interactions, such as for example, hemoglobin binding to its cognate ligand oxygen and binding of the λ phage *cl* repressor to its operator sites.

In some embodiments where the receptor component comprises more that one interaction site, the receptor component can comprise at least first and second interaction sites, wherein the first and second interaction sites are different. Different interaction sites refer to different portions of the receptor component that interact with the first binding component as compared to the interaction with the second binding component. Embodiments in which the receptor component comprises different interacting sites include, among others, proteins that are allosterically regulated and proteins that bind a regulatory ligand in order to bind to another binding component. Various examples of this type of binding will be apparent to the skilled artisan.

In some embodiments, the methods can be used to examine binding interactions in which the binding component and the receptor component are the same components. These binding interactions are homogenous binding interactions. Examples of homogeneous interactions include, by way of example and not limitation, dimerization of protein subunits or annealing of palindromic polynucleotide sequences.

In other embodiments, the methods can be used to examine binding interactions in which the binding component and the receptor components are different. These binding interaction are heterogeneous or heterologous interactions. Exemplary binding interactions of this type include, as examples, protein-DNA interactions, protein-small molecule interactions, DNA-small molecule interactions, and annealing of non-palindromic DNA sequences.

In some embodiments, the methods can be used to examine binding interactions in which the binding components comprise at least first and second binding components, wherein the first and second binding components are different. In various embodiments, the different first and second binding components can bind to the same binding sites, overlapping binding sites, or non-overlapping binding sites.

In embodiments where the binding of first and second binding component occurs at the same or overlapping interacting sites, the first and second binding components can bind the receptor component competitively. As used herein, "competitive binding" refer to at least first and second binding components in which their corresponding interaction sites overlap such that binding of the second binding component interferes with the binding of the first binding component. Increasing the concentration of one of the competitively binding components attenuates or eliminates the binding of the other competitively binding component. For example, in the context of enzyme-substrate interactions, a competitive inhibitor binds to the active site of the enzyme and inhibits binding of the substrate. Increasing substrate concentration, however, competes out the competitive inhibitor such that the enzyme parameter of maximal enzyme velocity $V_m$ is not affected while the apparent affinity for substrate $K_m$ is affected depending on the concentration of the competitive inhibitor.

In some embodiments, the first and second binding components can bind the receptor component non-competitively. As used herein, "non-competitive binding" refer to at least first and second binding components that bind to the receptor component in other than a competitive manner. Generally, in non-competitive interactions, the first binding component binds at an interaction site that does not overlap with the interaction site of the second binding component such that binding of the second binding component does not directly interfere with the binding of the first binding component. Thus, any effect on binding of the first binding component by the binding of the second binding component is through a mechanism other than direct interference with the binding of the first binding component. For example, in the context of enzyme-substrate interactions, a non-competitive inhibitor binds to a site other than the active site of the enzyme. As such, increasing the substrate concentration may not appreciably affect the enzyme parameters of $V_m$ and/or $K_m$ displayed in presence of the non-competitive inhibitor.

Non-competitive binding encompasses mixed-type interactions in which the first and second binding components bind to the receptor component with characteristics of both competitive and non-competitive binding, and also includes un-competitive binding interactions in which a second binding component binds at an interaction site that does not overlap with the binding of the first binding component but binds to the second interaction site only when the first interaction site is occupied by the first binding component. For example, in the context of enzyme-substrate interactions, an un-competitive inhibitor generally binds to the enzyme-substrate complex, thereby increasing the apparent substrate affinity $K_m$ (*i.e.*, substrate or product remains bound to enzyme longer) while decreasing the maximal enzyme activity $V_m$.

In some embodiments, the binding component comprises an agonist that binds to a receptor component. An "agonist" refers to a substance that binds to the receptor component and is capable of triggering a physiological action of the receptor component to which it binds. Thus, the binding of an agonist produces a biological action, while an antagonist, as further described below blocks the action of the agonist. For example, many drugs mimic the effects of an endogenous binding component by binding to the cognate receptor component and activating the signal transduction systems normally triggered by the endogenous binding component.

In some embodiments, the binding component comprises an antagonist that binds to a receptor component. An "antagonist" refers to a substance that binds to the receptor component and is capable of inhibiting or attenuating the physiological action of the receptor component to which it binds. Many drugs work by blocking the action of endogenous receptor agonists, such as for example hormones and neurotransmitters. Pharmacokinetic antagonists refer to drugs that alter the way a cell or organism reacts to another drug. Antagonists that compete with an agonist for a receptor are competitive antagonists while antagonists that antagonize by other means are non-competitive antagonists.

In some embodiments, the binding component comprises an inverse agonist. An "inverse agonist" refers to a binding component that binds to the same receptor component interaction site as an agonist for that receptor component but exerts the opposite biological or pharmacological effect. A characteristic of inverse agonist is inhibition by antagonists that also block agonist activity.

It is to be understood that binding interactions other than those expressly described can also be examined using the methods herein. Application of the methods to such binding interactions will be apparent to the skilled artisan.

2.2 Detecting Unbound and Bound Components

To determine the number of binding component bound per number of receptor component in the methods described herein, the unbound and bound components in the mixture are translocated through a pore. The term "pore" refers to any constriction or limited volume that restricts the passage of binding and receptor components. Pore includes apertures, holes, and channels. Channel includes, among others, trough, groove, or any conduit for passage of the components in the mixture to be detected in the detection region. The pore or channel dimensions can depend on the detection mode used. However, the size of the pore is at least such that it permits translocation of the unbound and bound components for detection. Thus the pore or channel is a nanopore having a diameter or channel dimension of about 100 nm or less, about 50 nm or less, about 20 nm or less, about 10 nm or less, about 5 nm or less, or about 2 nm or less, to about 0.5 nm. In some embodiments, the pore is of a dimension sufficient to limit the translocation through the pore to a single binding component, receptor component, and/or bound component.

The term "translocation" refers to movement of the component through the pore for detection in the detection region. In some embodiments, the translocation is directed translocation where a force is applied to move the component preferentially in a specified direction. The force can be any force, such as electromotive gradients, pressure gradients, concentration gradients, temperature gradients, osmotic gradients, or any other suitable force that can directionally transport the components in the mixture through the pore. Various modes for directional transport are further described below.

The term "detection region" refers to a region in which the unbound and/or bound components are detected. The detection region can be within the pore, juxtaposed on the pore, on the pore entrance or exit, or present through a portion or the entirety of the pore. Various configurations will depend on the detection mode used to detect the components in the binding mixture. Upon translocation through the pore, the unbound and any bound components are interrogated at the detection region to detect a detectable property associated with the unbound and bound components. Suitable detection modes include, by way of example and not limitation, charge-induced field effect, as further described below.

Detection of the detectable property of the unbound and bound components generates a signal pattern that identifies the detected component. This signal pattern associated with the detected component can be compared to a set of reference signal patterns to assist in correlating the measured signal pattern to a specific component in the mixture. Reference signal patterns can be obtained by analyzing the binding component and receptor component separately and then as a mixture to ascertain the characteristic or signature signal patterns associated with each component in the binding reaction.

Various detection modes capable of generating distinguishable signal patterns for each species of unbound component (*e.g.*, binding and receptor component) as well as each species of bound component can be used. Where the receptor component comprises more than one interaction site, a detection mode can be selected that can generate different signal patterns for each species of bound component, for example a receptor component with one bound binding component and a receptor component with two bound binding components. Furthermore, depending on the sensitivity of the detection, the signal pattern generated may permit distinguishing between binding to a first interaction site as compared to binding to a second interaction site. This may be possible in some embodiments where the first binding component is different from the second binding component such that independent binding events can be distinguished as well as complexes in which both binding components are bound to the receptor component. However, it is to be understood that analysis of the binding interaction need not require distinguishing between each species of unbound components or bound components. As an example, for an interaction involving a receptor with two interaction sites for the same binding component, an average equilibrium binding constant can be obtained by determining the total number of bound components without the need to distinguish between each species of bound component.

A variety of detection modes are applicable to the methods herein. In some examples, the detectable property is the effect of the translocated component on the electrical properties of the pore. Pore electrical properties include among others, current amplitude, impedance, duration, and frequency. Devices for detecting the pore's electrical properties typically comprise a pore incorporated into a thin film or a membrane, where the film or membrane separates a cis chamber and a trans chamber connected by a conducting bridge. The mixture to be analyzed is placed on the cis side of the pore in an aqueous solution, typically comprising one or more dissolved salts such as potassium chloride. Application of an electric field across the pore using electrodes positioned in the cis and trans side can be used to direct translocation of the components through the pore. The size and geometry of the component can affect the migration of ions through the pore, thereby altering the pore's electrical properties. Current is measured at a suitable time frequency to obtain sufficient data points to detect a current signal pattern. The generated signal pattern can then be compared to a set of reference patterns to identify the component being detected. Shifts in current amplitude, current duration, and current magnitude define a signal pattern for the specific component in the mixture. Measurement of current properties of a pore, such as by patch clamp techniques, is described in various reference works, for example, Hille, B, 2001, Ion Channels of Excitable Membranes, 3rd Ed., Sinauer Associates, Inc., Sunderland, MA.

In an example, the detected property is quantum tunneling of electrons. Quantum tunneling is the quantum-mechanical effect of transitioning through a classically-forbidden energy state via a particle's quantum wave properties. Electron tunneling generally occurs where a potential barrier exists for movement of electrons between a donor and an acceptor. To detect electron tunneling, a microfabricated electrode tip is positioned about 2 nanometers from the component to be detected. At an appropriate separation distance, electrons tunnel through the region between the tip and the component, and if a voltage is applied between the tip and the component, a net current of electrons (*i.e.*, tunneling current) flows through the gap in the direction of the voltage bias. Where the device uses detection electrodes for measuring tunneling current, the electrodes can be positioned proximately to the translocating components such that there is electron tunneling between the detection electrodes and translocated components of the mixture. As further discussed below, the arrangement of the electrodes relative to the direction of translocation can dictate the type of electron tunneling detected.

In an example, analysis of the binding interactions can involve detecting current flow occurring through the translocating component (*e.g.*, longitudinally along a nucleic acid chain) (Murphy et al., 1994, Proc Natl Acad Sci USA 91(12):5315-9). For detection of such electron tunneling, the detection electrodes are positioned longitudinally to the direction of translocation such that there is a gap between the electrodes parallel to the direction of translocation. In various embodiments, the detection electrodes can be placed on opposite sides of a layer(s) (*e.g.*, membrane) separating the two sides of the pore, while in other embodiments, the detection electrodes may be positioned within the layer(s) that separate the two sides of the pore.

Another mode of electron tunneling is that occurring across the component, *i.e.*, in a direction transverse to the direction of translocation through the pore. Differences in the chemical compositions, hydration structures, interactions with charged ions, spatial orientation of chemical groups in the component can alter the transverse electron transport characteristics, and thus provide a basis for distinguishing one component from another component in the binding mixture. For detection of electron tunneling across the pore (*e.g.,* transverse to an extended nucleic acid chain), the detection electrodes can be positioned on one side of the nanopore to interrogate the translocated component. For transverse detection, the tips of the detection electrodes can be dimensioned to interrogate a single component. In other embodiments, the dimensions of the detection electrodes can be arranged to interrogate larger or more than one component. For example, for the detection of polynucleotides, the electrodes can be dimensioned to interrogated about 2 or more, about 5 or more, about 10 or more, or about 20 bases or more depending on the resolution required to detect and distinguish the polynucleotide from other components in the binding mixture.

In the present invention, the detection technique is based on imaging charge-induced fields, as described in U.S. Patent No. 6,413,792 and U.S. published application No. 2003/0211502. Semiconductor devices for detection based on charge induced fields are also described in these references. Application of a voltage between a source region and a drain region results in flow of current from the source to the drain if a channel for current flow forms in the semiconductor. Because each component in the binding mixture can have an associated charge, passage of a component through the semiconductor pore can induce a change in the conductivity of the semiconductor material lining the pore, thereby inducing a current of a specified magnitude and waveform. Currents of differing magnitude and waveform can be produced by different components because of differences in charge, charge distribution, and size of the molecules. In the embodiments disclosed in U.S. Patent No. 6,413,792, a component passes through a pore formed of a p-type silicon layer. Translocation of the components can be achieved by methods similar to those used to move a component through other types of channels, as described herein. The magnitude of the charge-induced current is expected to be on the order of microampere range, which is higher than the picoampere currents expected for electron tunneling-based detection.

It is to be understood that although descriptions above relate to individual detection techniques, in some embodiments, a plurality of different techniques can be used to examine the binding mixture. Examples of multiple detection modes include, among others, current blockade in combination with electron tunneling current, and current blockage in combination with imaging charge induced fields. Concurrent detection with different detection modes can be used to identity a component in the binding reaction by correlating the detection time ofthe resulting signal obtained from different detection modes. Upon detection of the unbound and/or any bound components in the mixture, the relevant binding parameters can be determined as described above.

Various devices employing the various detection modes can be used for analyzing the binding mixture. These include, among others, biological based systems that employ a biological pore or channel embedded in a membrane and solid state systems in which the channel or pore is made whole or in part from a fabricated or sculpted solid state component, such as silicon. Devices using biological pores, such as α-hemolysin and porin, are described in Kasianowiscz et al., 1996, Proc Natl Acad Sci USA 93:13770-13773; Howorka et al., 2001, Nature Biotechnol. 18:1091-5; Szabo et al., 1998, FASEB J. 12:495-502; and U.S. Patent Nos. 5,795,782, 6,015,714, 6,267,872, and 6,428,959. In some embodiments, analysis of the binding reaction is carried out by translocating the components in the binding mixture through a pore fabricated from non-biological materials. Pores, including channels, can be made from a variety of solid state materials using a number of different techniques, including, among others, chemical deposition, electrochemical deposition, electroplating, electron beam sculpting, ion beam sculpting, nanolithography, chemical etching, laser ablation, and other methods well known in the art (see, *e.g.,* Li et al., 2001, Nature 412:166-169; and WO 2004/085609). Solid state materials include, by way of example and not limitation, any known semiconductor materials, insulating materials, and metals. Thus, the solid state pores can comprise without limitation silicon, silicon, silicon nitride, germanium, gallium arsenide, metals (*e.g.*, gold, silver, platinum), metal oxides, and metal colloids.

To prepare a pore of appropriate dimensions, various feedback procedures can be employed in the fabrication process. In embodiments where ions pass through a hole, detecting ion flow through the solid state material provides a way of measuring pore size generated during fabrication (see, *e.g.*, U.S. Published Application No. 2005/0126905). In other embodiments, where the electrodes define the size of the pore, electron tunneling current between the electrodes can provide information on the gap size. Increases in tunneling current indicate a decrease in the gap distance between the electrodes. Other feedback techniques will be apparent to the skilled artisan.

In some embodiments, the pore can be fabricated using ion beam sculpting, as described in Li et al., 2003, Nature Materials 2:611-615. In the described process, a layer of low stress silicon nitride film is deposited onto a silicon substrate via low pressure chemical vapor deposition. A combination of photolithography and chemical etching can be used to remove the silicon substrate to leave behind the silicon nitride layer. To form the pore, a focused ion beam (*e.g.*, argon ion beam of energy 0.5 to 5.0 KeV and diameter 0.1 to 0.5 mm) is used to generate a hole in the silicon nitride membrane. By suitable adjustment of the ion beam parameters (*e.g.*, total time the silicon nitride is exposed to the ion beam and the exposure duty cycle) and sample temperature, material can be either removed to enlarge the hole or material added to decrease the hole size. Ion beam bombardment at room temperature and low duty cycle results in migration of material into the hole while bombardment at 5°C and longer duty cycles results in enlargement of the hole. Measuring the amount of ions transmitted through the pore provides a feedback mechanism for precisely controlling the final pore size (Li et al., *supra*). To form a pore of appropriate dimensions, a hole larger than the final desired pore dimensions can be made using sculpting parameters that result in loss of the silicon nitride. Subsequently, the size of the pore can be adjusted to an appropriate dimension using sculpting parameters that result in movement of material into the initially formed hole.

In other embodiments, the pores can be made by a combination of electron beam lithography and high energy electron beam sculpting (see, *e.g.*, Storm et al., 2003, Nature Materials 2:537-540). A silicon-on-insulator, fabricated according to known methods, can be used to form a silicon membrane, which is then oxidized to form a silicon oxide layer. Using a combination of electron-beam lithography and anisotropic etching, the silicon oxide is removed to expose the silicon layer. Holes are made in the silicon by KOH wet etching and the silicon oxidized to form a silicon oxide layer of sufficient depth, such as for example a layer of about 40 nm. Exposure of the silicon dioxide to a high energy electron beam (*e.g.*, from a transmission electron microscope) deforms the silicon dioxide layer surrounding the hole. Whether the initial holes are enlarged or decreased depends on the initial size. Holes 50 nm or smaller appear to decrease in size while holes of about 80 nm or larger increase in size. A similar approach for generating a suitable pore by ion beam sputtering technique is described in Heng et al., 2004, Biophy J 87:2905-2911. In this technique, the pores are formed using lithography with a focused high energy electron beam on metal oxide semiconductor (CMOS) combined with general techniques for producing ultrathin films.

In other embodiments, the pore can be constructed as described in U.S. Patent No. 6,627,067; 6,464,842; 6,783,643; and U.S. Publication No. 2005/0006224 by sculpting of silicon nitride. Initially, a layer of silicon nitride is deposited on both sides of a silicon layer by chemical vapor deposition. Following addition of a photoresist in a manner that leaves a portion of the silicon nitride layer exposed, the exposed silicon nitride layer on one side is removed by conventional ion etching techniques to leave behind a silicon coated with silicon nitride on the other side. The silicon can be removed by any number of etching techniques, such as by anisotropic KOH etching, thus leaving behind a membrane of silicon nitride. The thickness of the silicon nitride membrane can be controlled by adjusting the thickness deposited onto the silicon. By use of electron beam lithography or photolithography, a cavity is produced on one side of the silicon nitride layer followed by thinning of the membrane on the other side of the cavity. Suitable thinning processes include, among others, ion beam sputtering, ion beam assisted etching, electron beam etching, and plasma reactive etching. Numerous variations on this fabrication process, for example, use of silicon nitride layer sandwiched between two silicon layers, can be used to generate different sized pores. As noted above, a feedback mechanism based on measuring the rate and/or intensity of ions passing through the pore provides a method of controlling the pore size during the fabrication process.

In other embodiments, the pore can be constructed as a gold or silver nanotube. In some embodiments, these pores are formed using a template of porous material, such as polycarbonate filters prepared using a track etch method, and depositing gold or other suitable metal on the surface of the porous material. Track etched polycarbonate membranes are typically formed by exposing a solid membrane material to high energy nuclear particles, which creates tracks in the membrane material. Chemical etching is then employed to convert the etched tracks to pores. The formed pores have a diameter of about 10 nm and larger. Adjusting the intensity of the nuclear particles controls the density of pores formed in the membrane. Nanotubes can be formed on the etched membrane by depositing a metal, typically gold or silver, into the track etched pores via an electroless plating method (Menon et al., 1995, Anal Chem 67:1920-1928). This metal deposition method uses a catalyst deposited on the surface of the pore material, which is then immersed into a solution containing Au(I) and a reducing agent. The reduction of Au(I) to metallic Au occurs on surfaces containing the catalyst. Amount of gold deposited is dependent on the incubation time such that increasing the incubation time decreases the inside diameter of the pores in the filter material. Thus, the pore size can be controlled by adjusting the amount of metal deposited on the pore. The resulting pore dimension is measured using various techniques, for instance, gas transport properties using simple diffusion or by measuring ion flow through the pores using patch clamp type systems. The support material is either left intact, or removed to leave gold nanotubes. Electroless plating technique is capable of forming pore sizes from less than about 1 nm to about 5 nm in diameter, or larger as required. Gold nanotubes having pore diameter of about 0.6 nm appears to distinguish between Ru(bpy)2+2 and methyl viologen, demonstrating selectivity of the gold nanopores (Jirage et al., 1997, Science 278:655-658). Modification of a gold nanotube surface is readily accomplished by attaching thiol containing compounds to the gold surface or by derivatizing the gold with other functional groups. This features permits attachment of pore modifying compounds. Devices, such as the cis/trans apparatuses used with the biological pores described herein, can also be used with the gold nanopores to analyze binding reactions.

Where the detection mode involves current flow through the translocated component (*e.g.*, electron tunneling current), the solid state membrane can be metalized by various techniques. A conductive layer can be deposited on both sides of the membrane to generate electrodes suitable for interrogating the components via longitudinal electron tunneling current. In some embodiments, the conductive layer can be deposited on one surface of the membrane to form electrodes suitable for interrogating components across the pore, for example, transverse electron tunneling current. Various methods for depositing conductive materials are known, including, sputter deposition (*i.e.*, physical vapor deposition), non-electrolytic deposition (*e.g.*, colloidal suspensions), and electrolytic deposition. Other metal deposition techniques include, among others, filament evaporation, metal layer evaporation, electron-beam evaporation, flash evaporation, and induction evaporation.

In some embodiments, the detection electrodes can be formed by sputter deposition, where an ion beam bombards a block of metal and vaporizes metal atoms, which are then deposited on a wafer material in the form of a thin film. Depending on the lithography method used, the metal films are then etched by means of reactive ion etching or polished using chemical-mechanical polishing. Metal films can be deposited on preformed pores or deposited prior to fabrication of the pore.

In some embodiments, the detection electrodes can be fabricated by electrodeposition (see, *e.g.*, Xiang et al., 2005, Angew. Chem. Int. Ed. 44:1265-1268; Li et al., Applied Physics Lett. 77(24):3995-3997; and U.S. application publication No. 2003/0141189). These fabrication process are suitable for generating a pore and corresponding detection electrodes positioned on one face of the solid state film, such as for detecting transverse electron tunneling. Initially, a conventional lithographic process is used to form a pair of facing electrodes on a silicon dioxide layer, which is supported on a silicon wafer. An electrolyte solution covers the electrodes, and metal ions are deposited on one of the electrodes by passing current through the electrode pair. Deposition of metal on the electrodes over time decreases the gap distance between the electrodes, creating not only detection electrodes but a dimensioned gap for translocation of the components in the binding reaction. The gap distance between the electrodes can be controlled by a number of feedback processes. In some configurations, the feedback for controlling the distance between the two electrodes can rely on the potential difference between the two electrodes. As the gap between the electrodes decreases, the potential difference decreases. In other configurations, control of the distance between the two electrodes uses the electron tunneling current across the electrode pair (Li et al, *supra*). As the distance between the electrodes decrease, electron tunneling current increases. Feedback control using electron tunneling is suitable for fabrication of electrodes with gap distances of about 1 nm or less, while the feedback control based on electrode gap potential allows fabrication of electrodes having gap distances about 1 to about 10 nm. Rate of electrodeposition depends on the electrolyte concentration and the current flowing through the electrodes. Constant current can be used to form layers of metal on the electrodes. In other embodiments, pulses of current can be used to deposit a known number of metal atoms onto the electrodes per each pulse cycle, and thereby provide precise control over electrode fabrication process.

Where the detection is based on imaging of charge induced field effects, a semiconductor device can be fabricated as described in U.S. Patent No. 6,413,792 and U.S. published application No. 2003/0211502. The methods of fabricating these detection devices can use techniques similar to those employed to fabricate other solid state pores. In some embodiments, the field effect detector is made using a silicon-on-insulator that comprises a silicon substrate with a silicon dioxide layer and a p-type silicon layer (doped silicon in which the majority of the charge carriers are positively charged holes). A shallow n-type silicon (doped silicon in which the majority of the charge carriers are negatively charged holes) layer is formed in the p-type silicon layer by ion implantation and addition of an n-type dopant, while another n-type silicon layer that extends through the p-type silicon layer is formed on another region of the silicon-on-insulator. Removal of the silicon substrate and silicon dioxde layers by etching exposes the p-type silicon on the face opposite to the first formed shallow n-type layer. On the newly exposed face of the p-type silicon, a second shallow n-type silicon layer is formed, which connects to the n-type silicon layer that extends through the p-type silicon layer. For analyzing the binding reaction, a pore that extends through the two shallow n-type silicon layers and the p-type silicon layer is generated by various techniques, for example by ion etching or lithography (*e.g.*, optical or electron beam). To decrease the pore size, a silicon dioxide layer can be formed by oxidizing the silicon. Metal layers are attached to the first formed n-type silicon layer and the n-type silicon layer that extends through p-type silicon, thereby forming the source and drain regions.

In the various embodiments herein for the analysis of the components in the mixture, the pore can be configured in various formats. In some embodiments, the device comprises a membrane, either biological or solid state, containing the pore held between two reservoirs, also referred to as cis and trans chambers (see, *e.g.*, U.S. Patent No. 6,627,067). A conduit for electron migration between the two chambers allows electrical contact of the two chambers, and a voltage bias between the two chambers can direct translocation of the binding component through the pore. A variation of this configuration is used in the analysis of current flow through biological nanopores, as described in U.S. Patent Nos. 6,015,714 and 6,428,959; and Kasianowiscz et al., 1996, Proc Natl Acad Sci USA 93:13770-13773.

Variations of the device above are also disclosed in U.S. application publication no. 2003/0141189. In these embodiments, a pair of nanoelectrodes fabricated by electrodeposition is positioned on a substrate surface. The electrodes face each other and have a gap distance sufficient for passage of the component to be analyzed. An insulating material protects the nanoelectrodes, exposing only the tips ofthe electrodes for purposes of detection. The insulating material and nanoelectrodes separate a chamber serving as a sample reservoir and a chamber to which the component to be analyzed is delivered by translocation. Cathode and anode electrodes provide an electric field for directing translocation from the sample chamber to the delivery chamber.

The current bias used to direct translocation through the pore can be generated by applying an electric field through the pore. In some embodiments, the electric field is a constant voltage or constant current bias. In other embodiments, the transloction of the components can be controlled through a pulsed operation of the electrophoresis electric field parameters (see, *e.g.*, U.S. Patent Application No. 2003/141189 and U.S. Patent No. 6,627,067). Pulses of current can provide a method of precise translocation for a defined time period through the pore and, in some instances, to briefly hold the component within the pore, and thereby provide greater resolution of the electrical properties of the component being analyzed.

The pore devices can further comprise an electric or electromagnetic field for restricting the orientation of the component as it passes through the pore. This holding field can be used to decrease the movement of the molecules within the pore. Movement of the component in the detection region can increase the background noise of the detected signal. For instance, when current blockade is measured, movement of a polymer within the pore is likely to result in variations of current flow. Similarly, where the detection measures electron tunneling current, the current signal is likely to be sensitive to the spatial orientation of the detected component relative to the detection electrodes. By holding or restricting the orientation of the molecule as it translocates through the nanopore, variations in the detected signal can be minimized.

In some embodiments, an electric field that is orthogonal to the direction of translocation can be used to restrict the movement of components, such as polymers, within the pore. This is illustrated in U.S. application publication No. 2003/0141189 through the use of two parallel conductive plates above and beneath the sample plate. These electrodes generate an electric field orthogonal to the direction of translocation, thus holding the component to one of the sample plates. For example, a negatively charged backbone of a DNA, or nucleic acid modified to have negative charges on one strand, can be oriented onto the anodic plate, thereby limiting the motion of the polynucleotide. Analogous use of an orthogonal electric field to hold a component in a limited orientation for detection is described in U.S. Patent No. 6,627,067. In this embodiment, electrodes positioned to generate an electric field orthogonal to the direction of translocation are used to hold the component in a groove, where the sample is interrogated with a probe (*e.g.*, electron tunneling probe). Similar to the control of the electric field for directing transport through a pore, the orthogonal electric field can be controlled in regard to the duration and amplitude of the holding field. The electric field used for translocation is coordinated with the electric field used to hold the component to be detected in a restricted orientation to precisely control translocation through the pore.

In some embodiments, controlling the position of the component in the pore can be carried out by the method described in U.S. application publication No. 2004/0149580, which employs an electromagnetic field created in the pore via a series of electrodes positioned near or on the pore. In these embodiments, one set of electrodes applies a direct current voltage and radio frequency potential while a second set of electrodes applies an opposite direct current voltage and a radio frequency potential that is phase shifted by 180 degrees with respect to the radio frequency potential generated by the first set of electrodes. This radio frequency quadrupole is expected to hold a changed particle (*e.g.*, a cell receptor) in the center of the field (*i.e.*, center of the pore). Holding the translocating component in the middle of the pore is predicted to reduce the variability of electron flow through a pore and may also provide consistency in current flow measured by electron tunneling. It is suggested that altering the amplitude of the radio frequency quadrupole could also be used to force an component to one side of the pore and thereby slow the rate of translocation.

2.3 Uses of the Methods of Analyzing Binding Interactions

As described herein, the methods can be used in analyzing binding interaction involving a variety of components. Thus, the receptor component can be any agent that can bind a binding component and vice versa.

In some embodiments, the binding component, receptor component, or both the binding and receptor component can comprise a small organic molecule. In various embodiments, small organic molecule can comprise any organic compound including, among others, alkyls, heteroalkyls, cycloalkyls, heterocycloalkyls, aryls, heteroaryls, ary-aryls, polycyclic aryls, fused ring systems, and bridged ring systems. Exemplary cycloalkyl compounds include, but are not limited to, cyclopropyl; cyclobutyls such as cyclobutanyl and cyclobutenyl; cyclopentyls such as cyclopentanyl and cyclopentenyl; cyclohexyls such as cyclohexanyl and cyclohexenyl based compounds. Exemplary heterocycloalkyl compounds can include, but are not limited to, tetrahydrofuranyl (*e.g.*, tetrahydrofuran-2-yl, tetrahydrofuran-3-yl, etc.), piperidinyl (*e.g.*, piperidin-1-yl, piperidin-2-yl, etc.), morpholinyl (*e.g.*, morpholin-3-yl, morpholin-4-yl, etc.), and piperazinyl (*e.g.*, piperazin-1-yl, piperazin-2-yl, etc.) based compounds.

Exemplary aryls can include, but are not limited to, compounds based on aceanthrylene, acenaphthylene, acephenanthrylene, anthracene, azulene, benzene, chrysene, coronene, fluoranthene, fluorene, hexacene, hexaphene, hexalene, *as*-indacene, s-indacene, indane, indene, naphthalene, octacene, octaphene, octalene, ovalene, penta-2,4-diene, pentacene, pentalene, pentaphene, perylene, phenalene, phenanthrene, picene, pleiadene, pyrene, pyranthrene, rubicene, triphenylene, trinaphthalene, and the like, as well as the various hydro isomers thereof. In some embodiments, the aryl compound comprises a $(C_5-C_{15})$ aryl, or $(C_5-C_{10})$ aryl. Exemplary aryl compounds can comprise cyclopentadienyl, phenyl and naphthyl.

Exemplary heteroaromatic compounds can comprise compounds based on, among others, acridine, benzimidazole, benzisoxazole, benzodioxan, benzodioxole, benzofuran, benzopyrone, benzothiadiazole, benzothiazole, benzotriazole, benzoxaxine, benzoxazole, benzoxazoline, carbazole, β-carboline, chromane, chromene, cinnoline, furan, imidazole, indazole, indole, indoline, indolizine, isobenzofuran, isochromene, isoindole, isoindoline, isoquinoline, isothiazole, isoxazole, naphthyridine, oxadiazole, oxazole, perimidine, phenanthridine, phenanthroline, phenazine, phthalazine, pteridine, purine, pyran, pyrazine, pyrazole, pyridazine, pyridine, pyrimidine, pyrrole, pyrrolizine, quinazoline, quinoline, quinolizine, quinoxaline, tetrazole, thiadiazole, thiazole, thiophene, triazole, and xanthene.

In various embodiments, the small organic molecule can comprise an organic compound of about 100 to about 4000 Daltons, about 200 to about 4000 Daltons, or about 500 to about 3000 Daltons. The molecule can be any type or class of compounds, including, among others, pesticides, hormones, vitamins, antibiotics, antiviral compounds, pharmaceutical and drug compounds, and enzyme inhibitors. Other small organic molecules suitable for the purposes herein will be apparent to the skilled artisan.

In some embodiments, the binding component, receptor component, or both the binding and receptor component can comprise a carbohydrate. As used herein, "carbohydrate" refers to compounds that contain oxygen, hydrogen, and carbon atoms and are related by the generic chemical formula $C_n(H_2O)_n$ or derivatives thereof. In various embodiments, the carbohydrate can be independent of other compounds or associated with other compounds, such as lipids, polypeptides, and nucleic acids. Carbohydrates can be monosaccharides, disaccharides, oligosaccharides, and polysaccharides. Monosaccharides can comprise aldoses, which have an aldehyde group on the first carbon atom, and ketoses, which typically have a ketone group on the second carbon. They can also be classified into trioses, tetroses, pentoses, hexoses, and so forth, depending on the number carbon atoms they contain. Exemplary monosaccharides include glyceraldehyde, erythose, threose, ribose, arabinose, xylose, lyxose, allose, altrose, glucose, mannose, gulose, idose, galactose, and talose.

In some embodiments, the carbohydrates can comprise disaccharides, which are composed of two monosaccharide units linked together by a covalent glycosidic bond. Common disaccharides include, among others, cellobiose, maltose, gentobiose, trehalose, sucrose. In other embodiments, the carbohydrate can comprise oligosaccharides and polysaccharides, which are composed of longer chains of monosaccharide units linked together by glycosidic bonds. The distinction between the two is based upon the number of monosaccharide units present in the chain. Oligosaccharides typically contain between three and nine monosaccharide units, and polysaccharides contain greater than ten monosaccharide units. Oligosaccharides are found as a common form of protein posttranslational modification of proteins. Polysaccharides include, among others, starch, cellulose, chitin and glycogen. Various receptor components that bind carbohydrates are known to the skilled artisan, and are further described below.

In some embodiments, the binding component, receptor component, or both the binding and receptor components can comprise a lipid or fatty acid. As used herein, a "lipid" refers to hydrocarbon based molecules that are non-polar or hydrophobic and is generally soluble in non-polar solvents. Lipids also include amphipathic or amphiphilic molecules, which are characterized by the presence of both hydrophobic and hydrophilic portions. Lipids can be acyclic or cyclic, straight or branched, saturated or unsaturated. Classes of lipids include, as examples, fatty acids, glycerides, and non-glycerides. Fatty acid refers to an organic acid with a hydrophobic portion, such as an aliphatic chain, and can be saturated or unsaturated. Exemplary saturated fatty acids include among others, butyric, lauric (dodecanoic acid), myristic (tetradecanoic acid), palmitic (hexadecanoic acid), stearic (octadecanoic acid), and arachidic (eicosanoic acid) fatty acids. Exemplary unsaturated fatty acids include, among others, alpha linolenic acid, docosahexaenoic acid, eicosapentaenoic acid, linoleic acid, arachidonic acid, oleic acid, and erucic acid. Unsaturated fatty acids can be in the cis or trans configuration.

Glycerides or glycerolipids refers to esters formed from glycerol and fatty acids. Glycerides can be in the form of monoglycerides, diglycerides, or triglyderides. Glycerides also encompass phosphoglycerides or glycerophospholipids, which comprise sn-glycero-3-phosphoric acid that contains at least one O-acyl, or O-alkyl or O-alk-1'-enyl residue attached to the glycerol moiety and a polar head made of a nitrogenous base, a glycerol, or an inositol unit. Non-glycerides include, among others, sphingolipids, sterols (*e.g.*, cholesterol, estrogen, and testosterone), prenols (*e.g.*, terpenoids), and polyketides. The lipids can be present independently of other molecules, or non-covalently or covalently attached to other molecules. For example, lipids can be present in the form of micelles or attached covalently to a carbohydrate or protein.

In some embodiments, the binding component, receptor component, or both binding and receptor components can comprise a polypeptide. As used herein, "polypeptide" is used interchangeably with the terms "peptide," "oligopeptide," and "protein" and refers to at least two amino acids connected by an amide linkage. The polypeptide can be of any length, linear or circular, or comprise branched structures, such as for example, ubiquitinated polypeptides. The terms "polypeptide," "peptide;" "oligopeptide," and "protein" includes those with D- and L-amino acids, and mixtures of D- and L-amino acids. The amino acids in the polypeptide can comprise genetically encoded amino acids, but also comprise, either in whole or in part, of naturally-occurring and/or synthetic non-encoded amino acids. Commonly encountered non-encoded amino acids include, but are not limited to, 2,3-diaminopropionic acid; ornithine; citrulline; homolysine; phosphoserine; phosphothreonine; homoaspartic acid; homoglutanic acid; homoalanine; norvaline; homoleucine, homovaline; homoisolencine; homoarginine; homocysteine; homoserine; hydroxyproline and homoproline. Additional non-encoded amino acids will be apparent to those of skill in the art (*see, e.g.,* the various amino acids provided in Fasman, 1989, CRC Practical Handbook of Biochemistry and Molecular Biology, CRC Press, Boca Raton, FL, at pp. 3-70 and the references cited therein).

In some embodiments, the binding component, receptor component, or both the binding and receptor component can comprise various polypeptide analogs. As used herein, "polypeptide analog" refers to any polypeptides in which the amide linkage is replaced with an isostere of an amide linkage or substituted amide linkages. In various embodiments, substituted amide linkages include, but are not limited to, groups of the formula -C(O)NR$^2$, where R$^2$ is (C$_1$-C$_6$) alkyl, (C$_5$-C$_{10}$) aryl, substituted (C$_5$-C$_{10}$) aryl, (C$_6$-C$_{16}$) arylalkyl, substituted (C$_6$-C$_{16}$) arylalkyl, 5-10 membered heteroaryl, substituted 5-10 membered heteroaryl, 6-16 membered heteroarylalkyl or substituted 6-16 membered heteroarylalkyl. In a specific embodiment, R$^2$ is (C$_1$-C$_6$) alkanyl, (C$_2$-C$_6$) alkenyl, (C$_2$-C$_6$) alkynyl or phenyl.

Isosteres of amides generally include, but are not limited to, -NR$^3$-SO-, -NR$^3$-S(O)$_2$-, -CH$_2$-CH$_2$-, -CH=CH- (*cis* and *trans*), -CH$_2$-NH-, -CH$_2$-S-, -CH$_2$-O-, -C(O)-CH$_2$-, -CH(OH)-CH$_2$- and -CH$_2$-S(O)$_2$-, where R$^3$ is hydrogen or R$^2$ and R$^2$ is as defined above. These interlinkages can be included in the polypeptides in one polarity or in the reverse polarity. Peptide analogs including such non-amide linkages, as well as methods of synthesizing such analogs, are well-known (see, *e.g.*, Spatola, 1983, "Peptide Backbone Modifications," In: Chemistry and Biochemistry of Amino Acids, Peptides and Proteins, Weinstein, Ed., Marcel Dekker, New York, pp. 267-357; Morley, 1980, Trends Pharm. Sci. 1:463-468; Hudson et al., 1979, Int. J. Prot. Res. 14:177-185 (-CH$_2$-NH-, -CH$_2$-CH$_2$); Spatola et al., 1986, Life Sci. 38:1243-1249; Spatola, 1983, "Peptide Backbone Modifications: the Ψ [CH2S] Moiety as an Amide Bond Replacement," In: Peptides: Structure and Function V, J. Hruby and D.H. Rich, Eds., Pierce Chemical Co., Rockford, IL, pp. 341-344 (-CH$_2$-S-); Hann, 1982, J. Chem. Soc. Parkin Trans. L 1:307-314 (-CH=CH-, *cis* and *trans*); Almquist et al., 1980, J. Med. Chem. 23:1392-1398 (-C(O)-CH$_2$-); European Patent Application EP 45665; Chemical Abstracts CA 97:39405 (-CH(OH)-CH$_2$-); Holladay et al., 1983, Tetrahedron Lett. 24:4401-4404 (-CH(OH)-CH$_2$-); and Hruby, 1982, Life Sci. 31:189-199 (-CH$_2$-S-).

Alternatively, one or more amide linkages may be replaced with peptidomimetic and/or amide mimetic moieties. Non-limiting examples of such moieties are described in Olson et al., 1993, J. Med. Chem. 36:3039-3049; Ripka & Rich, 1998, Curr. Opin. Chem. Biol. 2:441-452; Borchardt et al., 1997, Adv. Drug. Deliv. Rev. 27:235-256 and the various references cited therein.

In some embodiments, the polypeptides can comprise a retro peptide or retro peptide analog. A "retro peptide" or "retro peptide analog" refers to a peptide or peptide analog having a primary sequence that is the reverse (in the N+C direction) of the primary sequence of a corresponding parent peptide or peptide analog.

In some embodiments, the polypeptides can comprise an inverso peptide or inverso peptide analog. An "inverso peptide" or "inverso peptide analog" refers to a peptide or peptide analog having a primary sequence that is identical to that of a corresponding parent peptide or peptide analog, but in which all chiral α-carbons are in the opposite configuration.

In some embodiments, the polypeptides can comprise a retro-inverso peptide or retro-inverso peptide analog. A "retro-inverso peptide" or "retro-inverso peptide analog" refers to a peptide or peptide analog having the combined features of a retro peptide or retro peptide analog and an inverso peptide or inverso peptide analog, as defined above. Retro-inverso peptides and retro-inverso peptide analogs can be made either by reversing the polarity of the peptide or analogous bonds of a corresponding parent peptide or peptide analog or by reversing the order of the primary sequence (in the N+C direction) and changing the chirality of the α-carbons of a corresponding parent peptide or peptide analog.

Classes of polypeptides useful in the methods herein include, among others, enzymes, cellular receptors, DNA binding proteins, carbohydrate binding proteins, antibodies, lipid binding proteins, signal transduction proteins, peptide hormones, and peptide antibiotics. Other classes of polypeptides useful as binding and/or receptor components will be apparent to the skilled artisan.

In some embodiments, the binding component, receptor component, or both binding and receptor components can comprise a nucleobase polymer. As used herein, a "nucleobase polymer" or "nucleobase oligomer" refers to two or more nucleobases that are connected by linkages that permit the resultant nucleobase polymer or oligomer to hybridize to a polynucleotide having a complementary nucleobase sequence. Nucleobase polymers or oligomers include, but are not limited to, poly- and oligonucleotides (*e.g.*, DNA and RNA polymers and oligomers), poly- and oligonucleotide analogs and poly- and oligonucleotide mimics, such as polyamide or peptide nucleic acids. Nucleobase polymers or oligomers can vary in size from a few nucleobases, from 2 to 40 nucleobases, 10 to 25 nucleobases, 12 to 30 nucleobases, or 12 to 20 nucleobases, to several hundred nucleobases, to several thousand nucleobases, or more. "Nucleobase" or "base" refers to those naturally occurring and synthetic heterocyclic moieties commonly known to those who utilize nucleic acid or polynucleotide technology or utilize polyamide or peptide nucleic acid technology to thereby generate polymers that can hybridize to polynucleotides in a sequence-specific manner. Non-limiting examples of suitable nucleobases include: adenine, cytosine, guanine, thymine, uracil, 5-propynyl-uracil, 2-thio-5-propynyl-uracil, 5-methylcytosine, pseudoisocytosine, 2-thiouracil and 2-thiothymine, 2-aminopurine, N9-(2-amino-6-chloropurine), N9-(2,6-diaminopurine), hypoxanthine, isoguanine (iG), N9-(7-deaza-guanine), N9-(7-deaza-8-azaguanine) and N8-(7-deaza-8-aza-adenine). Other non-limiting examples of suitable nucleobases include those nucleobases illustrated in Figures 2(A) and 2(B) of Buchardt et al. (W0 92/20702 or W0 92/20703).

In some embodiments, the nucleobase polymer can comprise a polynucleotide or oligonucleotide. As used herein, "polynucleotide" or "oligonucleotide" refers to nucleobase polymer or oligomer in which the nucleobases are connected by sugar phosphate linkages (sugar-phosphate backbone). Exemplary poly- and oligonucleotides include polymers of 2'-deoxyribonucleotides (DNA) and polymers of ribonucleotides (RNA). A polynucleotide can be composed entirely of ribonucleotides, entirely of 2'-deoxyribonucleotides or combinations thereof.

In some embodiments, the nucleobase polymer can comprise a polynucleotide or oligonucleotide analog. As used herein, "polynucleotide or oligonucleotide analog" refers to nucleobase polymer or oligomer in which the nucleobases are connected by a sugar phosphate backbone comprising one or more sugar phosphate analogs. Typical sugar phosphate analogs include, but are not limited to, sugar alkylphosphonates, sugar phosphoramidites, sugar alkyl- or substituted alkylphosphotriesters, sugar phosphorothioates, sugar phosphorodithioates, sugar phosphates and sugar phosphate analogs in which the sugar is other than 2'-deoxyribose or ribose, nucleobase polymers having positively charged sugar-guanidyl interlinkages such as those described in U.S. Patent No. 6,013,785 and U.S. Patent No. 5,696,253 (see also, Dagani 1995, Chem Eng News 4-5:1153; Dempey et al., 1995, J Am Chem Soc 117:6140-6141). Such positively charged analogues in which the sugar is 2'-deoxyribose are referred to as "DNGs," whereas those in which the sugar is ribose are referred to as "RNGs." Specifically included within the definition of poly- and oligonucleotide analogs are locked nucleic acids (LNAs; see, *e.g.* Elayadi et al., 2002, Biochemistry 41:9973-9981; Koshkin et al., 1998, J Am Chem Soc 120:13252-3; Koshkin et al., 1998, Tetrahedron Letters 39:4381-4384; Jumar et al 1998, Bioorganic & Medicinal Chemistry Letters 8:2219-2222; Singh and Wengel, 1998, Chem Commun 12:1247-1248; WO 00/56746; WO 02/28875; and, WO 01/48190 ).

In some embodiments, the nucleobase polymer can comprise a polynucleotide or oligonucleotide mimic. As used herein "polynucleotide or oligonucleotide mimic" refers to a nucleobase polymer or oligomer in which one or more of the backbone sugar-phosphate linkages is replaced with a sugar-phosphate analog. Such mimics are capable of hybridizing to complementary polynucleotides or oligonucleotides, or polynucleotide or oligonucleotide analogs or to other polynucleotide or oligonucleotide mimics, and can include backbones comprising one or more of the following linkages: positively charged polyamide backbone with alkylamine side chains as described in U.S. Patent No. 5,786,461; U.S. Patent No. 5,766,855; U.S. Patent No. 5,719,262; U.S. Patent No. 5,539,082 and WO 98/03542 (see also, Haaima et al., 1996, Angewandte Chemie Int'l Ed. in English 35:1939-1942; Lesnick et al., 1997, Nucleosid. Nucleotid. 16: 1775-1779; D'Costa et al., 1999, Org Lett 1:1513-1516 see also Nielsen, 1999, Curr Opin Biotechnol 10:71-75); uncharged polyamide backbones as described in WO 92/20702 and U.S. Patent No. 5,539,082; uncharged morpholinophosphoramidate backbones as described in U.S. Patent No. 5,698,685, U.S. Patent No. 5,470,974, U.S. Patent No. 5,378,841 and U.S. Patent No. 5,185,144 (see also, Wages et al., 1997, BioTechniqures 23:1116-1121); peptide-based nucleic acid mimic backbones (see, *e.g.,* U.S. Patent No. 5,698,685); carbamate backbones (see, e.g., Stirchak & Summerton, 1987, J Org Chem 52:4202); amide backbones (see, *e.g.,* Lebreton, 1994, Synlett. 1994:137); methylhydroxyl amine backbones (see, *e.g.,* Vasseur et al., 1992, J Am Chem Soc 114:4006)*;* 3'-thioformacetal backbones (see, *e.g.,* Jones et al., 1993, J Org Chem 58:2983) and sulfamate backbones (see, *e.g.*, U.S. Patent No. 5,470,967).

In some embodiments, the nucleobase polymers can comprise a chimeric nucleobase polymer. A "chimeric nucleobase polymer" or "chimeric oligonucletide" refers to a nucleobase polymer or oligomer comprising a plurality of different polynucleotides, polynucleotide analogs, and polynucleotide mimics. For example, a chimeric oligo may comprise a sequence of DNA linked to a sequence of RNA. Other examples of chimeric oligos include a sequence of DNA linked to a sequence of PNA, and/or a sequence of RNA linked to a sequence of PNA.

Because any of the compound types above can be part of a binding interaction, various types of binding interactions can be examined using the methods herein. These include interactions between, among others, small molecule - small molecule; small molecule - polypeptide, small molecule - nucleobase polymer, polypeptide - polypeptide, polypeptide - nucleobase polymer, nucleobase polymer - nucleobase polymer, lipid-protein, and lipid-nucleic acid.

Examples of small molecule - small molecule interactions include, among others, the interaction of clathrates with their corresponding guest molecules. An exemplary clathrate is cyclodextrins, which are cyclic oligosaccharides generally composed of five or more glucopyranoside units. In some embodiments, cyclodextrins can contain glucose monomers ranging from six to eight units in a ring, such as $\alpha$-cyclodextrin, a six membered ring molecule; $\beta$-cyclodextrin, a seven membered ring molecule; and $\gamma$-cyclodextrin, an eight membered ring molecule. Cyclodextrans can bind other small organic molecules, including among others, insecticide trichlorfone; cholesterol and other sterols; and various lipophilic drugs (*e.g*., albendazole, mebendzole, digitoxin, ibuproxam, prioxicam, levenopamil, sulindac, and danazol). Another exemplary clathrate is crown ether, which generally are cyclic oligomer of ethylene oxide, *i.e.*, -$CH_2CH_2O$- and characterized by its ability to solvate cations through the oxygen atoms that coordinate with the cation in the inside portion of the ring. The size of the interior of the crown ether determines the size of the cation it can solvate such that an 18-crown-6 binds a potassium cation, a 15-crown-5 binds a sodium cation and 12-crown-4 binds a lithium cation.

In some embodiments, the methods can be used to analyze the binding interactions between a small molecule and a protein. Examples of small molecule - protein interactions include, among others, phorbol esters interactions with phorbol ester receptors; estrogen and estrogen analog interactions with estrogen receptors, testosterone interactions with androgen receptors, opiate interactions with opiate receptors, tetracycline or doxycline interactions with Tet controlled transactivator (tTA); mannose interactions with mannose binding protein; N-linked glycan interactions with concanavalin A; β-galactoside interaction with galectins; maltose interactions with maltose binding protein (MalE); arabinose interactions with arabinose binding protein, dopamine interactions with dopamine receptor, and serotonin interactions with 5-HT-receptor.

In some embodiments, the methods can be used to analyze the binding interactions between a small molecule and a nucleobase polymer. Examples of small molecules that interact with nucleobase polymers include, among others, DNA intercalators (*e.g.*, ethidium bromide, DAPY, aminoacridine, acridine orange, proflavine, daunomycin, actinomycin, YO, and YOYO, etc.); cis-platinum drugs (*e.g.*, cis-diamminedichloroplatinum), benzypyrene, topotecan, campothecin, netropsin, pyrrole-imidazole (Py-Im) polyamides, and chlorambucil. Other small molecules capable of interacting with nucleobase polymers will be apparent to the skilled artisan.

In some embodiments, the methods can be used to analyze polypeptide-polypeptide interactions. A variety of polypeptides are known to bind to other polypeptides, including binding to modified polypeptides, such as phosphorylated or lipidated polypeptides. In some embodiments, polypeptide - polypeptide interactions can occur through the interaction of protein interaction domains. Thus, the methods can be used to examine interactions between polypeptides with various protein - interaction domains and their cognate receptor domains. Exemplary protein - protein interaction domains include, by way of example and not limitation, SH2 domains (src homology domain 2), SH3 domain (src homology domain 3), PTB domain (phosphotyrosine binding domain), FHA domain (forkedhead associated domain), WW domain, 14-3-3 domain, pleckstrin horology domain, Cl domain, C2 domain, FYVE domain (Fab-1, YGL023, Vps27, and EEA1), death domain, death effector domain, caspase recruitment domain, Bcl-2 homology domain, bromo domain, chromatin organization modifier domain, F box domain, hect domain, ring domain ($Zn^{+2}$ finger binding domain), PDZ domain (PSD-95, discs large, and zona occludens domain), sterile a motif domain, ankyrin domain, arm domain (armadillo repeat motif), WD 40 domain and EF-hand (calretinin), and PUB domain (Suzuki T. et al., 2001, Biochem Biophys Res Commun 287: 1083-87).

In some embodiments, the methods can be used to analyze polypeptide - nucleobase polymer interactions. Examples of polypeptides that can interact with nucleobase polymers include, among others, peptide distamycin A, synthetic amphipathic peptides Ac-(Leu-Ala-Arg-Leu)3-NH-linker, and the 3 $_{10}$-helix Ac-(Aib-Leu-Arg)4-NH-linker containing the [beta]-loop-builder [alpha]-aminoisobutyric acid (Aib). In other embodiments, the proteins that interact with nucleobase polymers can comprise various polypeptides involving in regulating nucleic acid metabolism, such as for example, transcription activators, enhancer binding proteins, transcription inhibitors, chromosome modeling proteins, DNA replication proteins, etc. Similar to protein - protein interaction domains, many nucleobase polymer binding proteins interact with the polymer through specific domains of common structure and function. Exemplary interaction domains include, by way of example and not limitation, helix-turn-helix domain, helix-loop-helix, leucine zipper domain, homeo box domain, $Zn^{+2}$ finger domain, paired domain, LIM domain, ETS domain, and T Box domain. Exemplary polynucleotide binding proteins include, among others, GAL-4, λ Cro protein, Jun/Fos complex, GCN-4, CREB, retinoid-X-receptor, retinoid receptor, vitamin D receptor, TFIIA, krupple, Antp, Ubx, myc, myb, NF-kb, Stat proteins (*e.g.*, Stat 1, State 2, Stat 3. Stat 4, Stat 5, etc.), MADS box proteins, TATA binding proteins, retinoblastoma protein, histones (*e.g.*, H1 (H5), H2A, H2B H3, and H4), mismatch binding protein (*e.g.*, Cel I), topoisomerases, uvrABC endonuclease, photoreactivating enzyme, single stranded binding protein (ssb), and recA. Other DNA binding proteins applicable to the methods herein will be apparent to the skilled artisan.

In some embodiments, the methods can be used to analyze nucleobase polymer-nucleobase polymer interactions. As discussed above, nucleobase polymer includes polynucleotides, polynucleotide analogs, and polynucleotide mimetics. In some embodiments where the receptor component comprises a polynucleotide, such as an oligonucleotide, the binding component can comprise a substantially complementary oligonucleotide or polynucleotide. As used herein, the term "substantially complementary" refers to a nucleobase polymer sequence capable of specifically hybridizing to a complementary sequence under conditions used to anneal or hybridize the nucleobase polymer. As used herein "annealing" or "hybridization" refers to the base-pairing interactions of one nucleobase polymer with another that results in the formation of a double-stranded structure, a triplex structure or other structures formed between nucleobase polymers through base pairing interactions. Annealing or hybridization can occur via Watson-Crick base-pairing interactions, but can be mediated by other hydrogen-bonding interactions, such as Hoogsteen base pairing.

In some embodiments, the annealing characteristics of a nucleobase polymer can be determined by the $T_m$ of the hybrid complex. The greater the $T_m$ value, the more stable the hybrid. $T_m$ is the temperature at which 50% of a nucleobase oligomer and its perfect complement form a double-stranded oligomer structure. The $T_m$ for a selected nucleobase polymer also varies with factors that influence or affect hybridization. For example, such factors include, but are not limited to, factors commonly used to impose or control stringency of hybridization, (*i.e.*, formamide concentration (or other chemical denaturant reagent), salt concentration (*i.e.*, ionic strength), hybridization temperature, detergent concentration, pH and the presence or absence of chaotropes. Optimal stringency for forming a hybrid combination can be found by the well-known technique of fixing several of the aforementioned stringency factors and then determining the effect of varying a single stringency factor. The same stringency factors can be modulated to control the stringency of hybridization of a PNA to a scaffold, except that the hybridization of a PNA is fairly independent of ionic strength. Optimal or suitable stringency conditions can be experimentally determined by examination of each stringency factor until the desired degree of discrimination is achieved.

The $T_m$ values for the nucleobase oligomers can be calculated using known methods for predicting melting temperatures (see, *e.g.*, Baldino et al., Methods Enzymology 168:761-777; Bolton et al., 1962, Proc. Natl. Acad. Sci. USA 48:1390; Bresslauer et al., 1986, Proc. Natl. Acad. Sci USA 83:8893-8897; Freier et al., 1986, Proc. Natl. Acad. Sci USA 83:9373-9377; Kierzek et al., Biochemistry 25:7840-7846; Rychlik et al., 1990, Nucleic Acids Res 18:6409-6412; Sambrook et al., 2001, Molecular Cloning: A Laboratoy Manual, 3rd Ed, Cold Spring Harbor Laboratory Press, Cold Spring Harbor, NY; Suggs et al., 1981, In Developmental Biology Using Purified Genes (Brown et al., eds.), pp. 683-693, Academic Press; and Wetmur, 1991, Crit Rev Biochem Mol Biol 26:227-259 ).

In some embodiments, the substantially complementary oligonucleotide or polynucleotide can comprise at least one nucleotide mismatch with the receptor oligonucleotide or polynucleotide. The methods herein can be used to examine the annealing of the substantially complementary polynucleotide with the nucleotide mismatch to the receptor polynucleotide. In some embodiments, depending on the length of the substantially complementary region, the number of nucleotide mismatches examined can comprise 2 or more nucleotide mismatches, about 5 nucleotide mismatches, about 10 or more nucleotide mismatches, or about 20 nucleotide mismatches or more. The number of nucleotide mismatches can comprise up to at least about 0.5%, about 1%, about 2%, about 5%, about 10%, about 20% up to about 30% of the nucleotide residues in the substantially complementary region.

In some embodiments, the annealing characteristics of a substantially complementary oligonucleotide or polynucleotide to a corresponding receptor oligonucleotide or polynucleotide can be examined at various annealing conditions. Parameters, such as for example, the ionic strength, chaotropic agents, hydrogen bond disruptors (*e.g.*, formamide, urea), pH, G/C content, and the temperature can be varied to ascertain the annealing behavior, such as for determining the optimal conditions for detecting a mismatch. Such analysis can be useful in choosing a particular oligonucleotide as a probe to detect a genetically heritable disorder attributable to a specific nucleotide sequence change in a specified gene.

In some embodiments, the methods can be used to analyze binding interactions involving lipids and proteins that bind the lipid. Exemplary lipid-protein interactions include, among others, monoacylated lipid interaction with lipid transfer proteins (Douliez et al., 2001, Eur J Biochem 268, 384-388); fatty acids, retinoids, and other hydrophobic ligand interaction with adipocyte lipid binding proteins (ALBP); fatty acids and eicosanoid interaction with fatty acid binding proteins (FABP); fatty acid CoA ester interaction with acyl-CoA-binding proteins (ACBP); phospholipid interaction with phospholipid binding proteins (*e.g.*, copine); and phosphatidylinositol interaction with phosphatidylinositol transfter proteins.

In some embodiments, the methods can be used to analyze lipid-nucleic acid interactions. Generally, these include cationic lipids in the form of liposomes or micelles that act as carriers for polynucleotides. Liposomes can be formed from various lipids and lipid combinations, including, among others, phosphatidylinositol, phosphatidylethanolamine, phosphatidylserine, dimethyl dioctadecyl ammonium bromide, dioleoyl phosphatidylethanolamine, dioleoylphosphatidylcholine, dipalmitoylphosphatidyl choline, and cholesterol. Other lipids for forming liposomes include, by way of example and not limitation, cationic lipids D282, D378, D383, D3886, D3897 and D3899, (Molecular Probes, Eugene, Oregon, USA).

In other embodiments, the methods can be used to analyze binding interactions involving an antibody and an antigen bound by the antibody. An "antigen" refers to any molecule or molecular group, including small organic molecules, carbohydrates, lipids/fatty acids, polypeptides, and nucleobase polymers recognized by at least one antibody. An antigen comprises at least one epitope or determinant capable of being recognized by the antibody. An "antibody" refers to immunological binding agent An "antibody" refers to a glycoprotein comprising at least two heavy (H) chains and two light (L) chains interconnected by disulfide bonds, or an antigen binding portion thereof. Each heavy chain is comprised of a heavy chain variable region (abbreviated herein as $V_H$) and a heavy chain constant region. The IgG heavy chain constant region is comprised of four domains, $C_{H1}$, hinge, $C_{H2}$ and $C_{H3}$. Each light chain is comprised of a light chain variable region (abbreviated herein as $V_L$) and a light chain constant region. The light chain constant region is comprised of one domain, $C_L$. The $V_H$ and $V_L$ regions can be further subdivided into regions of hypervariability, termed complementarity determining regions (CDR), interspersed with regions that are more conserved, termed framework regions (FR). Each VH and $V_L$ is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies can mediate the binding of the immunoglobulin to host tissues or factors, including various cells of the immune system (*e.g.*, effector cells) and the first component (Clq) of the classical complement system. Antibody includes antibodies of different isotypes, including those of the IgG, IgM, IgD, and IgA isotypes, and any antigen binding fragment (*i.e.*, "antigen-binding portion") or single chain thereof, and fragments, such as Fab and (Fab)$_2$ fragments.

Antibodies can be polyclonal or monoclonal. As used herein "monoclonal antibody" or "monoclonal antibody composition" refers to a preparation of antibody molecules of single molecular composition. A monoclonal antibody composition generally displays a single binding specificity and affinity for a particular epitope. The term "human monoclonal antibody" refers to antibodies displaying a single binding specificity which have variable and constant regions derived from human germline immunoglobulin sequences.

Antibodies can also be single chain Fv ("sFv") antibody, which is a covalently linked $V_H::V_L$ heterodimer. These single chain antibodies can be expressed from a gene fusion including $V_H$- and $V_L$-encoding genes linked by a peptide-encoding linker (see, e.g., Huston et al., 1988, Proc. Nat. Acad. Sci. USA 85(16):5879-5883). A number of methods have been described to discern chemical structures for converting the naturally aggregated but chemically separated light and heavy antibody chains from an antibody V region into an sFv molecule which will fold into a three dimensional structure substantially similar to the structure of an antigen-binding site (see, *e.g.*, U.S. Patent Nos. 5,091,513; 5,132,405; and U.S. Patent No. 4,946,778).

Examination of the antibody-antigen binding interactions using the methods herein can provide information as to the strength of binding, and in some instances, the portion of the antigen bound. In some embodiments, the antibody analyzed can be directed to any cellular molecule, such as for purposes of diagnostics or treatment of a disorder (*e.g.*, anti-TNF-$\alpha$, anti-Her-2, anti-CTLA-4, etc.).

In other embodiments, the methods can be used to examine an enzyme and its interaction with a binding component, such as a substrate, inhibitor, or an activator. The term "enzyme" refers to molecules or molecular aggregates that are capable of catalyzing chemical and biological reactions, and includes, without limitation, polypeptidies, peptides, RNAs, DNAs, ribozymes, antibodies and other molecules that can promote catalysis of a chemical or biological reaction. Exemplary enzymes useful for analysis can include, by way of example and not limitation, angiotensin converting enzyme, DNA polymerases, reverse transcriptases, proteases, esterases, kinases (*e.g.*, tyrosine, serine, threonine), helicases, topoisomerases, telomerases, nucleases, phosphatases, isomerases, and oxidoreductases. Various substrates, inhibitors, and activator for such enzymes will be apparent to the skilled artisan based on the enzyme chosen for analysis.

In some embodiments, the binding component can be a member of a library of binding components, and the methods can be used to examine the binding interactions of each member of the library. "Library of binding components" refers to a plurality of different binding components that can be screened for-the ability to interact with a receptor component. By identifying binding components displaying the optimal binding characteristics, for example equilibrium dissociation constant, association rate, and dissociation rate, a binding component useful in affecting the properties of the receptor component can be identified.

Preparation of libraries of compounds is well known to those of skill in the art. Such combinatorial chemical libraries include, but are not limited to, peptide libraries (see, *e.g.*, U.S. Patent No. 5,010,175, Furka, 1991, Pept. Prot. Res. 37:487-493, Houghton et al., 1991, Nature, 354:84-88, peptoids (PCT Publication No WO 91/19735), encoded peptides (PCT Publication WO 93/20242), random bio-oligomers (PCT Publication WO 92/00091), benzodiazepines (U.S. Pat. No. 5,288,514), diversomers such as hydantoins, benzodiazepines and dipeptides (Hobbs et al., 1993, Proc Nat Acad Sci USA 9010:6909-6913), vinylogous polypeptides (Hagihara et al., 1992, J. Amer. Chem. Soc. 114:6568), nonpeptidal peptidomimetics (Hirschmann et al., 1992, JAmer Chem Soc 11415 :9217-9218), oligocarbamates (Cho, et al., 1993, Science 261:1303), peptidyl phosphonates (Campbell et al., 1994, J Org Chem 59:658*; Gordon et al., 1994, JMed Chem 37:1385), nucleic acid libraries (Sambrook et al., *supra),* peptide nucleic acid libraries (see, *e.g.,* U.S. Patent 5,539,083), antibody libraries (see, *e.g.*, Vaughn et al., 1996, Nature Biotechnology 14(3)20 :309-314), and PCT/US96/10287), carbohydrate libraries (see, *e.g.*, Liang et al., 1996, Science 274:1520-1522, and U.S. Patent No. 5,593,853), and small organic molecule libraries (see, *e.g.*, isoprenoids, U.S. Patent No. 5,569,588; thiazolidinones and metathiazanones, U.S. Patent No. 5,549,974; pyrrolidines, U.S. Patent Nos. 5,525,735 and 5,519,134; morpholino compounds, U.S. Patent No. 5,506,337; benzodiazepines, U.S. Patent No. 5,288,514).

In the event that any definition or usage of a word or phrase used herein is in conflict with the definition and/or usage of that word or phrase in any other document the definition and/or usage of said word or phrase herein shall always control.

**Claims**

1. A method of analyzing binding interactions in solution, comprising: (a) contacting one or more binding components with a receptor component, wherein unbound and any bound components are capable of translocating through a nanopore; (b) detecting the unbound and any bound components by translocating the unbound and bound components through the pore, wherein the detecting is by imaging the charge induced field effect; and (c) determining the number of binding component bound per number of receptor component.

2. The method of claim 1 in which the unbound and any bound components are counted.

3. The method of claim 1 in which the receptor component has n interaction sites with the binding component, in which n is 2 to 10 and in which at least two of the interaction sites display cooperative interactions.

4.  The method of claim 1 in which the receptor component has n interaction sites with the binding component, in which n is 2 to 10 and in which the interaction sites comprise at least a first and second interaction sites, wherein the first and second interactions sites are different.

5.  The method of claim 1 in which the binding component and the receptor component are the same or the binding component and receptor component are different.

6.  The method of claim 1 in which the binding components comprise at least a first and second binding components, wherein the first and second binding components are different.

7.  The method of claim 6 in which the first and second binding components bind the receptor component competitively or bind the receptor component non-competitively.

8.  The method of claim 1 in which the receptor component comprises a protein that binds the binding component.

9.  The method of claim 8 in which the protein comprises an enzyme or a cellular receptor.

10. The method of claim 8 in which the binding component comprises an agonist of the protein or an antagonist of the protein.

11. The method of claim 8 in which the protein comprises an antibody.

12. The method of claim 11 in which the binding component comprises an antigen bound by the antibody.

13. The method of claim 1 in which the receptor component comprises an oligonucleotide.

14. The method of claim 13 in which the binding component comprises a protein that binds the oligonucleotide.

15. The method of claim 13 in which the binding component comprises a substantially complementary oligonucleotide.


**Patentansprüche**

1.  Verfahren zur Analyse von Bindungswechselwirkungen in einer Lösung, umfassend: (a) In-Kontakt-Bringen von einem oder von mehreren Bindungskomponenten mit einer Rezeptorkomponente, wobei ungebundene und alle gebundenen Komponenten dazu in der Lage sind, sich durch eine Nanopore zu verlagern; (b) Nachweis der ungebundenen und aller gebundenen Komponenten durch die Verlagerung der ungebundenen und der gebundenen Komponenten durch die Pore, wobei der Nachweis durch die Bildgebung das ladungsinduzierten Feldeffekts erfolgt, und (c) Bestimmung der Anzahl von Bindungskomponenten, die pro Anzahl von Rezeptorkomponenten gebunden sind.

2.  Verfahren nach Anspruch 1, wobei die ungebundenen und gebundenen Komponenten gezählt werden.

3.  Verfahren nach Anspruch 1, wobei der Rezeptor n Wechselwirkungsstellen mit der Bindungskomponente aufweist, wobei n gleich 2 bis 10 ist, und wobei mindestens zwei der Wechselwirkungsstellen kooperative Wechselwirkungen anzeigen,

4.  Verfahren nach Anspruch 1, wobei der Rezeptor n Wechselwirkungsstellen mit der Bindungskomponente aufweist, wobei n gleich 2 bis 10 ist, und wobei die Wechselwirkungsstellen mindestens eine erste und eine zweite Wechselwirkungsstelle umfassen, wobei die erste und die zweite Wechselwirkungsstelle verschieden sind.

5.  Verfahren nach Anspruch 1, wobei die Bindungskomponente und die Rezeptorkomponente gleich sind oder die Bindungskomponente und die Rezeptorkomponente verschieden sind.

6.  Verfahren nach Anspruch 1, wobei die Bindungskomponenten mindestens eine erste und eine zweite Bindungskomponente umfassen, wobei die erste und die zweite Bindungskomponente verschieden sind.

**7.** Verfahren nach Anspruch 6, wobei die erste und die zweite Bindungskomponente die Rezeptorkomponente konkurrierend binden oder die Rezeptorkomponente nicht konkurrierend binden.

**8.** Verfahren nach Anspruch 1, wobei die Rezeptorkomponente ein Protein umfasst, dass die Bindungskomponente bindet.

**9.** Verfahren nach Anspruch 8, wobei das Protein ein Enzym oder einen Zellrezeptor umfasst.

**10.** Verfahren nach Anspruch 8, wobei die Bindungskomponente einen Agonisten des Proteins oder einen Antagonisten des Proteins umfasst.

**11.** Verfahren nach Anspruch 8, wobei das Protein einen Antikörper umfasst.

**12.** Verfahren nach Anspruch 11, wobei die Bindungskomponente ein Antigen umfasst, das vom Antikörper gebunden ist.

**13.** Verfahren nach Anspruch 1, wobei die Rezeptorkomponente ein Oligonukleotid umfasst.

**14.** Verfahren nach Anspruch 13, wobei die Bindungskomponente ein Protein umfasst, das das Oligonukleotid bindet.

**15.** Verfahren nach Anspruch 13, wobei die Bindungskomponente ein im Wesentlichen komplementäres Oligonukleotid umfasst.

**Revendications**

**1.** Procédé d'analyse d'interactions de liaison en solution, comprenant :

(a) mettre en contact un ou plusieurs composants de liaison avec un composant récepteur, des composants non liés et tous composants liés étant capables de translocation à travers un nanopore ;
(b) détecter les composants non liés et tous composants liés par translocation des composants non liés et liés à travers le pore, la détection s'effectuant par imagerie de l'effet de champ induit par charge ; et
(c) déterminer le nombre de composant de liaison liés par nombre de composant récepteur.

**2.** Procédé selon la revendication 1, dans lequel les composants non liés et tous composants liés sont comptés.

**3.** Procédé selon la revendication 1, dans lequel le composant récepteur a n sites d'interaction avec le composant de liaison, dans lequel n est 2 à 10 et dans lequel au moins deux des sites d'interaction présentent des interactions coopératives.

**4.** Procédé selon la revendication 1, dans lequel le composant récepteur a n sites d'interaction avec le composant de liaison, dans lequel n est 2 à 10 et dans lequel les sites d'interaction comprennent au moins un premier et un second site d'interaction, les premier et second sites d'interaction étant différents.

**5.** Procédé selon la revendication 1, dans lequel le composant de liaison et le composant récepteur sont identiques ou le composant de liaison et le composant récepteur sont différents.

**6.** Procédé selon la revendication 1, dans lequel les composants de liaison comprennent au moins un premier et un second composant de liaison, les premier et second composants de liaison étant différents.

**7.** Procédé selon la revendication 6, dans lequel les premier et second composants de liaison lient le composant récepteur par compétition ou lient le composant récepteur non par compétition.

**8.** Procédé selon la revendication 1, dans lequel le composant récepteur comprend une protéine qui lie le composant de liaison.

**9.** Procédé selon la revendication 8, dans lequel la protéine comprend une enzyme ou un récepteur cellulaire.

**10.** Procédé selon la revendication 8, dans lequel le composant de liaison comprend un agoniste de la protéine ou un antagoniste de la protéine.

**11.** Procédé selon la revendication 8, dans lequel la protéine comprend un anticorps.

**12.** Procédé selon la revendication 11, dans lequel le composant de liaison comprend un antigène lié par l'anticorps.

**13.** Procédé selon la revendication 1, dans lequel le composant récepteur comprend un oligonucléotide.

**14.** Procédé selon la revendication 13, dans lequel le composant de liaison comprend une protéine qui lie l'oligonucléotide.

**15.** Procédé selon la revendication 13, dans lequel le composant de liaison comprend un oligonucléotide sensiblement complémentaire.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6413792 B
- US 20030211502 A
- US 5795782 A
- US 6015714 A
- US 6267872 B
- US 6428959 B
- WO 2004085609 A
- US 20050126905 A
- US 6627067 B
- US 6464842 B
- US 6783643 B
- US 20050006224 A
- US 20030141189 A
- US 6428959 A
- US 2003141189 A
- EP 45665 A
- WO 9220702 A
- WO 9220703 A
- US 6013785 A
- US 5696253 A
- WO 0056746 A
- WO 0228875 A
- WO 0148190 A
- US 5786461 A
- US 5766855 A

- US 5719262 A
- US 5539082 A
- WO 9803542 A
- US 5698685 A
- US 5470974 A
- US 5378841 A
- US 5185144 A
- US 5470967 A
- US 5091513 A
- US 5132405 A
- US 4946778 A
- US 5010175 A
- WO 9119735 A
- WO 9320242 A
- WO 9200091 A
- US 5288514 A
- US 5539083 A
- US 9610287 W
- US 5593853 A
- US 5569588 A
- US 5549974 A
- US 5525735 A
- US 5519134 A
- US 5506337 A

**Non-patent literature cited in the description**

- **MYSZKA, D.** *Methods Enzymol.,* 2000, vol. 323, 325-340
- **HILLE, B.** Ion Channels of Excitable Membranes. Sinauer Associates, Inc, 2001
- **MURPHY et al.** *Proc Natl Acad Sci USA,* 1994, vol. 91 (12), 5315-9
- **KASIANOWISCZ et al.** *Proc Natl Acad Sci USA,* 1996, vol. 93, 13770-13773
- **HOWORKA et al.** *Nature Biotechnol.,* 2001, vol. 18, 1091-5
- **SZABO et al.** *FASEB J.,* 1998, vol. 12, 495-502
- **LI et al.** *Nature,* 2001, vol. 412, 166-169
- **LI et al.** *Nature Materials,* 2003, vol. 2, 611-615
- **STORM et al.** *Nature Materials,* 2003, vol. 2, 537-540
- **HENG et al.** *Biophy J,* 2004, vol. 87, 2905-2911

- **MENON et al.** *Anal Chem,* 1995, vol. 67, 1920-1928
- **JIRAGE et al.** *Science,* 1997, vol. 278, 655-658
- **XIANG et al.** *Angew. Chem. Int. Ed.,* 2005, vol. 44, 1265-1268
- **LI et al.** *Applied Physics Lett.,* vol. 77 (24), 3995-3997
- **FASMAN.** CRC Practical Handbook of Biochemistry and Molecular Biology. CRC Press, 1989, 3-70
- Peptide Backbone Modifications. **SPATOLA.** Chemistry and Biochemistry of Amino Acids, Peptides and Proteins. Marcel Dekker, 1983, 267-357
- **MORLEY.** *Trends Pharm. Sci.,* 1980, vol. 1, 463-468
- **HUDSON et al.** *Int. J. Prot. Res.,* 1979, vol. 14, 177-185
- **SPATOLA et al.** *Life Sci.,* 1986, vol. 38, 1243-1249

- Peptide Backbone Modifications: the Ψ [CH2S] Moiety as an Amide Bond Replacement. **SPATOLA.** Peptides: Structure and Function. Pierce Chemical Co, 1983, 341-344
- **HANN.** *J. Chem. Soc. Parkin Trans. L,* 1982, vol. 1, 307-314
- **ALMQUIST et al.** *J. Med. Chem.,* 1980, vol. 23, 1392-1398
- **HOLLADAY et al.** *Tetrahedron Lett.,* 1983, vol. 24, 4401-4404
- **HRUBY.** *Life Sci.,* 1982, vol. 31, 189-199
- **OLSON et al.** *J. Med. Chem.,* 1993, vol. 36, 3039-3049
- **RIPKA ; RICH.** *Curr. Opin. Chem. Biol.,* 1998, vol. 2, 441-452
- **BORCHARDT et al.** *Adv. Drug. Deliv. Rev.,* 1997, vol. 27, 235-256
- **DAGANI.** *Chem Eng News,* 1995, vol. 4-5, 1153
- **DEMPEY et al.** *J Am Chem Soc,* 1995, vol. 117, 6140-6141
- **ELAYADI et al.** *Biochemistry,* 2002, vol. 41, 9973-9981
- **KOSHKIN et al.** *J Am Chem Soc,* 1998, vol. 120, 13252-3
- **KOSHKIN et al.** *Tetrahedron Letters,* 1998, vol. 39, 4381-4384
- **JUMAR et al.** *Bioorganic & Medicinal Chemistry Letters,* 1998, vol. 8, 2219-2222
- **SINGH ; WENGEL.** *Chem Commun,* 1998, vol. 12, 1247-1248
- **HAAIMA et al.** *Angewandte Chemie Int'l Ed. in English,* 1996, vol. 35, 1939-1942
- **LESNICK et al.** *Nucleosid. Nucleotid.,* 1997, vol. 16, 1775-1779
- **D'COSTA et al.** *Org Lett,* 1999, vol. 1, 1513-1516
- **NIELSEN.** *Curr Opin Biotechnol,* 1999, vol. 10, 71-75
- **WAGES et al.** *BioTechniqures,* 1997, vol. 23, 1116-1121
- **STIRCHAK ; SUMMERTON.** *J Org Chem,* 1987, vol. 52, 4202
- **LEBRETON.** *Synlett,* 1994, 137
- **VASSEUR et al.** *J Am Chem Soc,* 1992, vol. 114, 4006
- **JONES et al.** *J Org Chem,* 1993, vol. 58, 2983
- **SUZUKI T. et al.** *Biochem Biophys Res Commun,* 2001, vol. 287, 1083-87
- **BALDINO et al.** *Methods Enzymology,* vol. 168, 761-777
- **BOLTON et al.** *Proc. Natl. Acad. Sci. USA,* 1962, vol. 48, 1390
- **BRESSLAUER et al.** *Proc. Natl. Acad. Sci USA,* 1986, vol. 83, 8893-8897
- **FREIER et al.** *Proc. Natl. Acad. Sci USA,* 1986, vol. 83, 9373-9377
- **KIERZEK et al.** *Biochemistry,* vol. 25, 7840-7846
- **RYCHLIK et al.** *Nucleic Acids Res,* 1990, vol. 18, 6409-6412
- **SAMBROOK et al.** Molecular Cloning: A Laboratoy Manual. Cold Spring Harbor Laboratory Press, 2001
- **SUGGS et al.** Developmental Biology Using Purified Genes. Academic Press, 1981, 683-693
- **WETMUR.** *Crit Rev Biochem Mol Biol,* 1991, vol. 26, 227-259
- **DOULIEZ et al.** *Eur J Biochem,* 2001, vol. 268, 384-388
- **HUSTON et al.** *Proc. Nat. Acad. Sci. USA,* 1988, vol. 85 (16), 5879-5883
- **FURKA.** *Pept. Prot. Res.,* 1991, vol. 37, 487-493
- **HOUGHTON et al.** *Nature,* 1991, vol. 354, 84-88
- **HOBBS et al.** *Proc Nat Acad Sci USA,* 1993, vol. 9010, 6909-6913
- **HAGIHARA et al.** *J. Amer. Chem. Soc.,* 1992, vol. 114, 6568
- **HIRSCHMANN et al.** *J Amer Chem Soc,* 1992, vol. 11415, 9217-9218
- **CHO et al.** *Science,* 1993, vol. 261, 1303
- **CAMPBELL et al.** *J Org Chem,* 1994, vol. 59, 658
- **GORDON et al.** *J Med Chem,* 1994, vol. 37, 1385
- **VAUGHN et al.** *Nature Biotechnology,* 1996, vol. 14 (3), 309-314
- **LIANG et al.** *Science,* 1996, vol. 274, 1520-1522